# EUROPEAN PATENT SPECIFICATION

(11) **EP 0 641 206 B1**
(45) Date of publication and mention of the grant of the patent: **24.10.2001**
(21) Application number: 93911342.9
(22) Date of filing: 17.05.1993
(51) Int. Cl.: A61K 31/465, A61P 25/34

(54) **USE OF COTININE TO ALLEVIATE TOBACCO WITHDRAWAL SYNDROME**
VERWENDUNG VON COTININ ZUR LINDERUNG DES TABAKENTZUGSSYNDROM
UTILISATION DE LA COTININE POUR CALMER LE SYNDROME DE SEVRAGE DU TABAC

(30) Priority: 18.05.1992 US 885314; 21.10.1992 US 964277
(43) Date of publication of application: 08.03.1995
(73) Proprietor: PHARMACO BEHAVIORAL ASSOCIATES, INC., Bloomington, MN 55420 (US)
(72) Inventor: KEENAN, Robert M., Baltimore, MD 21224 (US); HATSUKAMI, Dorothy K., Golden Valley, MN 55422 (US)
(74) Representative: Maiwald, Walter, Dr. Dipl.-Chem.
(86) International application number: US9304672
(87) International publication number: WO9323045

(56) References cited:
- FR-M- 2 428
- US-A- 3 870 794
- CLIN.PHARM.THER. vol. 34, 1988, pages 604 - 11 'Cotinine disposition and effects' cited in the application
- CHEMICAL ABSTRACTS, vol. 55, no. 17, 1961, Columbus, Ohio, US; 'Tobacco products containing nicotine antagonists' column 16920 ;

## Description

Cigarette smoking continues to be the major preventable cause of death in the United States resulting in nearly 400,000 deaths per year due to cancer and heart disease. Despite the potential adverse health effects, the vast majority of cigarette smokers are unable to cease smoking.

The lack of smoking cessation success is thought to be related to the tobacco withdrawal syndrome or tobacco abstinence syndrome that most smokers experience during their attempts to quit. See, Office of Smoking and Health, The Health Consequences of Smoking: Nicotine Addiction. A Report to the Surgeon General, U.S. Govt. Print. Off., Washington D.C., DHHS Pub. No. (CDC) 88-8406 (1988). The most common effects are similar to those in almost all abstinence syndromes, and include decreased heart rate, anxiety, difficulty concentrating, impatience, irritability and restlessness. See, American Psychiatric Assoc., Diagnostic and Statistical Manual, Washington D.C. (3rd ed. 1980) at pages 159-160, 176-178. Most withdrawal effects occur within 24 hours, peak in the first 1-2 weeks and significantly decrease at one month. It is widely believed that the effects of abstinence from tobacco are due to nicotine deprivation, and that abstinence effects from smoking prevent smokers from stopping. See, J.R. Hughes et al., in Research and Advances in Alcohol and Drug Problems, Vol. 10, L.T. Kozlowski et al., eds., Plenum Pub. Corp. (1990) at pages 317-398.

Of the pharmacological approaches to aiding cessation of smoking, nicotine replacement, e.g., via transdermal nicotine patches or nicotine gum is the most widely used. Nicotine gum decreases abstinence discomfort, especially anxiety, decreased memory and irritability. On the other hand, nicotine gum does not reliably decrease weight gain or craving. Also, discontinuing use of nicotine gum leads to some of the same symptoms as the cigarette withdrawal syndrome. Furthermore, nicotine is toxic, and the availability of nicotine gum or patches poses a risk of poisoning to children and pets.

Other studies have demonstrated that alpha-2 agonists, such as clonidine, decrease postcessation anxiety, irritability and difficulty concentrating. Decreased sympathetic activity has been postulated to be the mechanism by which these drugs decrease abstinence effects. Although tobacco abstinence has some effects that could be attributed to sympathetic activity, it lacks the typical signs and symptoms of sympathetic overactivity, such as tachycardia, diaphoresis and hypertension. Thus, the mechanism by which alpha-2 agonists exert their effects is unclear. While a number of other pharmacological treatments, such as use of doxepin, ACTH, and corticotrophins, for abstinence symptoms have been tested, none of the studies reported baseline and postcessation values for abstinence symptoms. See, for example, S.J. Bourne (U.S. Patent No. 4,621,074).

Therefore, a continuing need exists for pharmacological treatments that will facilitate smoking cessation, e.g., by blocking or relieving tobacco withdrawal syndrome, or reducing the symptoms of nicotine withdrawal.

### SUMMARY OF THE INVENTION

The present invention provides various uses of cotinine, namely:

The use of cotinine or a pharmaceutically acceptable salt thereof to prepare a medicament effective to reduce or eliminate at least one of the symptoms of nicotine withdrawal or of tobacco withdrawal syndrome in a human, wherein the medicament is a unit dosage form containing from 30 mg to 1000 mg active ingredient, calculated as (-)-cotinine in the free base form.

The use of cotinine or a pharmaceutically acceptable salt thereof to prepare a medicament effective to reduce or eliminate at least one of the symptoms of nicotine withdrawal or of tobacco withdrawal syndrome in a human, wherein the medicament is in a form for administration in an amount of from 1.0 to 100 mg/kg human body weight/day, of active ingredient, calculated as (-)-cotinine in the free base form.

The use of cotinine or a pharmaceutically acceptable salt thereof to prepare a medicament effective to maintain abstinence from, or assist cessation of smoking or nicotine use, wherein the medicament is a unit dosage form containing from 30 mg to 1000 mg active ingredient, calculated as (-)-cotinine in the free base form.

The use of cotinine or a pharmaceutically acceptable salt thereof to prepare a medicament effective to maintain abstinence from, or assist cessation of smoking or nicotine use, wherein the medicament is in a form for administration in an amount of from 1.0 to 100 mg/kg human body weight/day, of active ingredient, calculated as (-)-cotinine in the free base form.

The present invention is exemplified by a study in which (-)-cotinine base was intravenously administered to abstinent cigarette smokers. The cotinine administration caused many subjective changes without significantly altering cardiovascular activity. While cotinine administration appeared to mildly exacerbate some of the symptoms of the tobacco withdrawal syndrome such as anxiety, restlessness and the overall tobacco withdrawal syndrome checklist (TWSC) subtotal score, it simultaneously decreased various symptoms of the TWS. Cotinine also reduced the overall craving for cigarettes, tobacco and/or nicotine experienced during the session.

Cotinine has many qualities which can enhance its value as a smoking cessation aid. Cotinine has a long in vivo half-life, complete oral bioavailability, minimal effect on the cardiovascular system, and has not been reported to be harmful even at very high doses in many species including man. Also, because cotinine has no significant effect on the heart, a combined pharmacologic treatment approach using cotinine and nicotine may be possible.

### DETAILED DESCRIPTION OF THE INVENTION

### Cotinine

Cotinine (1-methyl-5-(3-pyridinyl)-2-pyrrolidinone) has the formula shown below:

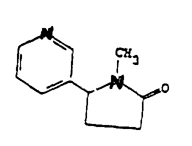

The physiologically active form is the (-)-isomer, so as used herein, the term "cotinine" includes (-)-cotinine, or the racemic form, (t)-cotinine. The free base, depicted above, can be employed in the practice of the invention, as can the pharmaceutically acceptable salts. These include the amine-acid addition salts of nontoxic organic acids or inorganic acids, such as the tartarate, fumarate ("scotine"), citrate, maleate, malate, hydrobromide, hydrochloride, sulfate, phosphate and the like. For example, see F. Vaitekunas, J. Amer. Chem. Soc., 79, 149 (1957). E.R. Bowman et al., in J. Pharmacol. and Exp. Ther., 135, 306 (1962) report the preparation of (-)-cotinine free base from (-)-nicotine. The preparation and purification of (-)-cotinine fumarate is described by N.L. Benowitz et al., Clin. Pharmacol. Ther., 34, 604 (1983).

Cotinine is the major metabolite of nicotine which accumulates in the body as a result of nicotine exposure and has previously been believed to be pharmacologically inactive. For example, see N.L. Benowitz, "The use of biologic fluid samples in assessing tobacco smoke consumption", in Measurement in the Analysis and Treatment of Smoking Behavior, J. Grabowski et al. eds., NIDA Research Monograph No. 48, U.S. DHHS, PHS, ADAMHA (1983). In contrast to nicotine, cotinine has a relatively long terminal elimination half-life (two versus sixteen hours, respectively). Due to this pharmacological characteristic, cotinine has become the principally used objective biochemical marker of nicotine exposure in cigarette smoking and/or cessation-related research paradigms.

While cotinine is a well-known metabolite of nicotine and is routinely measured in many laboratories, no systematic investigation of the physiological and subjective effects produced by intravenous cotinine administration has been performed in humans. K.I. Yamamoto et al., International J. Neuropharmacol., 4, 359 (1965) reported that intravenous cotinine produced increases in EEG activity and behavioral arousal in cats with only a slight decrease in blood pressure. In squirrel monkeys, intramuscular cotinine injections increased rates of responding on fixed interval schedules of reinforcement over a wide range of doses (M.E. Risner et al., J. Pharmacol. and Exp. Ther., 234, 113 (1985); S.R. Goldberg et al., Psychopharmacology, 97, 295 (1989)). These findings, taken together, suggest that cotinine is behaviorally active. However, the pharmacologic mechanism of action has yet to be determined.

In two recent human studies, the pharmacokinetic profiles of intravenous and orally administered cotinine were examined without emphasis on measuring the subjective and/or physiological changes induced by this compound (N.L. Benowitz et al., Clin. Pharmacol. and Therapeutics, 34, 604 (1983); P.J. DeSchepper et al., Eur. J. Pharmacol., 31, 583 (1987)). Moreover, using an uncontrolled experimental design, Benowitz et al., Clin. Pharm. and Ther., 34, 604 (1988), found that intravenous cotinine infusion over 60 min. produced no cardiovascular changes and significant decreases in subjective ratings of desire to smoke, irritability, low energy and anxiety/tension. These changes were comparable to placebo-induced changes found in other experiments with nicotine. Using a rapid infusion of cotinine over 5 minutes, no significant changes in the subjective ratings were observed. Consequently, Benowitz and his colleagues concluded that cotinine lacked significant pharmacologic activity in humans.

### Administration and Dosages

While it is possible that, for use in therapy, cotinine and/or its salts may be administered as the pure chemicals, as by inhalation of a fine powder via an insufflator, it is preferable to present the active ingredient as a pharmaceutical formulation. The invention thus further uses a pharmaceutical formulation comprising cotinine and/or a pharmaceutically acceptable salt thereof, together with one or more pharmaceutically acceptable carriers therefor and, optionally, other therapeutic and/or prophylactic ingredients. The carrier(s) must be 'acceptable' in the sense of being compatible with the other ingredients of the formulation and not deleterious to the recipient thereof.

Pharmaceutical formulations include those suitable for oral or parenteral (including intramuscular, subcutaneous and intravenous) administration. Forms suitable for parenteral administration also include forms suitable for administration by inhalation or insufflation or for nasal, or topical (including buccal, rectal, vaginal and sublingual) administration. The formulations may, where appropriate, be conveniently presented in discrete unit dosage forms and may be prepared by any of the methods well known in the art of pharmacy. Such methods include the step of bringing into association the active compound with liquid carriers, solid matrices, semi-solid carriers, finely divided solid carriers or combinations thereof, and then, if necessary, shaping the product into the desired delivery system.

Pharmaceutical formulations suitable for oral administration may be presented as discrete unit dosage forms such as hard or soft gelatin capsules, cachets or tablets each containing a predetermined amount of the active ingredient; as a powder or as granules; as a solution, a suspension or as an emulsion; or in a chewable base such as a synthetic resin or chicle for ingestion of the cotinine from a chewing gum. The active ingredient may also be presented as a bolus, electuary or paste. Tablets and capsules for oral administration may contain conventional excipients such as binding agents, fillers, lubricants, disintegrants, or wetting agents. The tablets may be coated according to methods well known in the art, i.e., with enteric coatings.

Oral liquid preparations may be in the form of, for example, aqueous or oily suspensions, solutions, emulsions, syrups or elixirs, or may be presented as a dry product for constitution with water or other suitable vehicle before use. Such liquid preparations may contain conventional additives such as suspending agents, emulsifying agents, non-aqueous vehicles (which may include edible oils), or preservatives.

The compounds used in the invention may also be formulated for parenteral administration (e.g., by injection, for example, bolus injection or continuous infusion) and may be presented in unit dose form in ampules, pre-filled syringes, small volume infusion containers or in multi-dose containers with an added preservative. The compositions may take such forms as suspensions, solutions, or emulsions in oily or aqueous vehicles, and may contain formulatory agents such as suspending, stabilizing and/or dispersing agents. Alternatively, the active ingredient may be in powder form, obtained by aseptic isolation of sterile solid or by lyophilization from solution, for constitution with a suitable vehicle, e.g., sterile, pyrogen-free water, before use.

For topical administration to the epidermis, the cotinine may be formulated as ointments, creams or lotions, or as the active ingredient of a transdermal patch. Suitable transdermal delivery systems are disclosed, for example, in A. Fisher et al. (U.S. Patent No. 4,788,603), or R. Bawa et al. (U.S. Patent Nos. 4,931,279, 4,668,506 and 4,713,224). Ointments and creams may, for example, be formulated with an aqueous or oily base with the addition of suitable thickening and/or gelling agents. Lotions may be formulated with an aqueous or oily base and will in general also contain one or more emulsifying agents, stabilizing agents, dispersing agents, suspending agents, thickening agents, or coloring agents. The active ingredient can also be delivered via iontophoresis, e.g., as disclosed in U.S. Patent Nos. 4,140,122, 4,383,529, or 4,051,842.

Formulations suitable for topical administration in the mouth include unit dosage forms such as lozenges comprising active ingredient in a flavored base, usually sucrose and acadia or tragacanth; pastilles comprising the active ingredient in an inert base such as gelatin and glycerin or sucrose and acacia; mucoadherent gels, and mouthwashes comprising the active ingredient in a suitable liquid carrier.

When desired, the above-described formulations can be adapted to give sustained release of the active ingredient employed, e.g., by combination with certain hydrophilic polymer matrices, e.g., comprising natural gels, synthetic polymer gels or mixtures thereof.

Pharmaceutical formulations suitable for rectal administration wherein the carrier is a solid are most preferably presented as unit dose suppositories. Suitable carriers include cocoa butter and other materials commonly used in the art, and the suppositories may be conveniently formed by admixture of the active compound with the softened or melted carrier(s) followed by chilling and shaping in molds.

Formulations suitable for vaginal administration may be presented as pessaries, tampons, creams, gels, pastes, foams or sprays containing, in addition to the active ingredient, such carriers as are known in the art to be appropriate.

For administration by inhalation, the compounds according to the invention are conveniently delivered from an insufflator, nebulizer or a pressurized pack or other convenient means of delivering an aerosol spray. Pressurized packs may comprise a suitable propellant such as dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount.

Alternatively, for administration by inhalation or insufflation, the compounds according to the invention may take the form of a dry powder composition, for example, a powder mix of the compound and a suitable powder base such as lactose or starch. The powder composition may be presented in unit dosage form in, for example, capsules or cartridges or, e.g., gelatin or blister packs from which the powder may be administered with the aid of an inhalator or insufflator.

For intra-nasal administration, the compounds of the invention may be administered via a liquid spray, such as via a plastic bottle atomizer. Typical of these are the Mistometer® (Wintrop) and the Medihaler® (Riker).

For topical administration to the eye, the cotinine can be administered as drops, gels (see, S. Chrai et al., U.S. Patent No. 4,255,415), gums (see S.-L. Lin et al., U.S. Patent No. 4,136,177) or via a prolonged-release ocular insert (see A.S. Michaels, U.S. Patent No. 3,867,519 and H.M. Haddad et al., U.S. Patent No. 3,870,791).

The pharmaceutical compositions used in the invention may also contain other adjuvants such as flavorings, colorings, antimicrobial agents, or preservatives.

It will be further appreciated that the amount of cotinine, or an active salt or derivative thereof, required for use in treatment will vary not only with the particular salt selected but also with the route of administration, the nature of the condition being treated and the age and condition of the patient and will be ultimately at the discretion of the attendant physician or clinician.

In general, however, a suitable dose will be in the range of from 1 to 100 mg/kg, e.g., from 10 to 75 mg/kg of body weight per day, such as 3 to about 50 mg per kilogram body weight of the recipient per day, preferably in the range of 6 to 90 mg/kg/day, most preferably in the range of 15 to 60 mg/kg/day, calculated as (-)-cotinine in the free base form.

The compound is conveniently administered in unit dosage form; for example, containing 30 to 1000 mg, conveniently 30 to 750 mg, most conveniently, 50 to 500 mg of active ingredient per unit dosage form.

Ideally, the active ingredient should be administered to achieve peak plasma concentrations of the active compound of from 0.5 to 75 µM, preferably, 1 to 50 µM, most preferably, 2 to 30 µM. This may be achieved, for example, by the intravenous injection of a 0.05 to 5% solution of the active ingredient, optionally in saline, or orally administered as a bolus containing about 1-100 mg of the active ingredient. Desirable blood levels may be maintained by continuous infusion to provide 0.01-5.0 mg/kg/hr or by intermittent infusions containing 0.4-15 mg/kg of the active ingredient(s).

The desired dose may conveniently be presented in a single dose or as divided doses administered at appropriate intervals, for example, as two, three, four or more sub-doses per day. The sub-dose itself may be further divided, e.g., into a number of discrete loosely spaced administrations; such as multiple inhalations from an insufflator or by application of a plurality of drops into the eye.

The invention will be further described by reference to the following detailed Example.

### Example I - Intravenous Administration of (-)-Cotinine

A. Subjects: Participants included 18 healthy male volunteers between the ages of 18 and 40 years old who had 1) no history of psychiatric, alcohol and drug abuse disorders, 2) smoked at least one pack of cigarettes per day for one year prior to study admission, 3) an expired-air carbon monoxide concentration of greater than 20 ppm, 4) not currently on any medication, and 5) not donated blood in the past 90 days. Potential subjects were carefully screened for physical and mental health problems.
B. Drug Preparation and Administration Procedures: (-)-Cotinine base was synthesized from (-)-nicotine using the bromine-zinc oxidation method described by E.R. Bowman et al., Biochem. Preparations, 10, 36 (1963). Hereinafter, the term "cotinine" will be used to refer to (-)-cotinine. The cotinine base was analyzed for impurities using gas chromatography/mass spectrometry and thin layer chromatography and found to be pure. Using sterile techniques, cotinine solution was prepared for intravenous administration. Cotinine base was combined with sterile normal saline solution to achieve a concentration of three mg of cotinine base per one ml of solution. This solution was autoclaved and found to be non-pyrogenic using a standard pyrogenicity testing. The cotinine solution was again tested for molecular structure integrity and concentration accuracy. Next, 10 ml of cotinine solution (30 mg cotinine) were placed into 20 ml injection vials, sealed and stored in a refrigerator until used. The placebo was ten ml of sterile normal saline solution. Placebo and active drug vials were prepared and labeled in a double-blind manner by pharmacy personnel. In addition to pharmacy personnel, one study physician who had no contact with subjects during the experimental sessions had access to the drug code in the event of a medical emergency.
During sessions, subjects received 10 ml (30 mg) of cotinine base solution diluted to 15 ml with sterile normal saline solution or placebo (15 ml of sterile saline solution) infused intravenously through a 20 gauge indwelling intravenous catheter. This infusion rate was chosen so as not to exceed two mg per minute of cotinine delivered to the subject. Infusions were performed using a controlled-rate syringe infusion pump. All subjects received cotinine and placebo infusions using a randomly assigned double-blind counterbalanced- order design.
C. Dependent Measures: The physiological parameters monitored included heart rate, systolic, diastolic and mean arterial blood pressure, and a 12-lead electrocardiogram (ECG) with measurement of PR, QRS and QT intervals. The biochemical parameters included expired-air carbon monoxide level (CO), serum nicotine and cotinine concentrations. Carbon monoxide was measured using standard techniques. The serum nicotine and cotinine concentration assays were performed using gas chromatography and mass spectrometry at the Laboratory of Physiological Hygiene at the University of Minnesota Medical School. See N.L. Benowitz, NIDA Research Monograph No. 48, US DHHS, PHS, ADAMHA (1983).
Self-reported ratings of subjective state, mood, cigarette withdrawal symptoms and adverse feelings were obtained from the subjects. These measures included the Profile of Mood States questionnaire (POMS), several 100 mm visual analog scales (VAS) and the tobacco withdrawal symptoms checklist (TWSC) of symptoms related to the cigarette withdrawal syndrome (J.R. Hughes et al., Archives of General Psychology, 43, 289, (1986)). The Record of Withdrawal Symptom is a 0=(none) - 5=(severe) scale of 12 symptoms associated with TWS: craving, irritable/angry, anxious/tense, difficulty concentrating, restless, impatient, excessive hunger, insomnia, increased eating, drowsiness, headaches and somatic symptoms (a miscellaneous group including tremor, heart racing, sweating, dizzy or g.i. problems).
Two 100 mm VAS forms were used. One with 10 adjectives including "Pleasant", "Need for Cigarettes", "Energy", "Hungry", "Down", "Sedated", "Anxious", "Stimulated", "Fatigue", "Craving for Cigarettes" and a separate VAS for "Craving for Tobacco" rated from "none" to "extremely." From the VAS forms, a measure of "vigor" was created by subtracting the sedation score from the stimulation score. Also, an adverse effects questionnaire (AEQ) was used to assess possible problems associated with cotinine administration. These problems were restlessness, headaches, tachycardia/palpitations, tremor, excessive sweating, nausea/vomiting, upset stomach, lightheadedness/dizzy, drowsy, irritable, and excessive salivation. The symptoms assessed were those known to be experienced following nicotine administration.
D. Procedure: This study was performed on an outpatient basis over nine days. Subjects were required to attend five sessions. All sessions were held at the Tobacco Research Laboratory associated with the University of Minnesota Hospital Complex, Minneapolis, MN. The first session was used to obtain informed consent, physical and psychological screening of the prospective participant, background and baseline data collection. Also, the subject habituated to the data collection procedures to be utilized during the sessions. If the participant met inclusion criteria, the participant was scheduled for his next visit. Prior to session 2, the subject was randomly assigned to one of the two drug administration order conditions.
Sessions 2 and 4 were used to collect data for all dependent variables under conditions of *ad libitum* cigarette smoking and serving as a baseline from which to assess tobacco withdrawal induced changes measured at the beginning of sessions 3 and 5, respectively. All sessions were scheduled to begin between 5 and 7 p.m. Sessions 2 and 4 were held seven days apart and lasted approximately 15 minutes. During these sessions, vital signs, CO, TWSC, VAS, POMS and AEQ were completed. Blood was drawn for later measurement of serum nicotine and cotinine concentration. At the end of sessions 2 and 4 and after departing the laboratory, subjects were required to refrain from cigarette smoking and other forms of tobacco use over the next 48 hours. At the end of this 48-hour period following sessions 2 and 4, subjects reported to the laboratory for the two-drug-infusion sessions 3 and 5, respectively.
During sessions 3 and 5, subjects received cotinine and placebo infusions in a counterbalanced order. Sessions 3 and 5 were held 48 hours after sessions 2 and 4 during which time the subject was tobacco abstinent as ascertained by using biochemical markers of smoke exposure, CO and serum cotinine concentrations. After the subject reported to the laboratory, baseline measurements of CO, vital signs, TWSC, VAS, POMS and AEQ were made. Next, the ECG electrodes were attached to the chest wall and limbs. For intravenous drug administration and access in the event of an adverse event, a 20 gauge indwelling catheter was placed in a prominent vein in the non-dominant forearm. This allowed the subject to freely complete subjective effects questionnaires during the remainder of the session. Heart rate and blood pressure were recorded. Using standard venipuncture techniques, five mls of blood were drawn from the antecubital area of the dominant arm for later serum nicotine and cotinine concentration analyses. At intervals of 5, 15, 30, 60 and 120 minutes after the drug infusion, heart rate, blood pressure, ECG, TWSC, VAS, and AEQ were completed, and blood was drawn for later serum nicotine and cotinine concentration analyses. Also, the POMS was completed at 30, 60 and 120 minutes after drug administration. The blood samples were allowed to stand for 30 minutes, centrifuged for 10 minutes and the serum was pipetted into plastic cryovials for storage in a -20°C freezer until the nicotine/cotinine assays were performed.
E. Statistical Analyses: All questionnaires were scored and entered into a computer by a blinded research assistant. At the end of the experiment and when all data scoring, collation and entry were completed, the drug code and serum cotinine concentrations were entered into the computer.
Of the eighteen subjects who began the study, fourteen subjects were considered to be tobacco abstinent using the biochemical markers of cigarette smoke exposure during the two abstinence periods. As a result, only the data from these fourteen individuals were included in the statistical analyses. The statistical analyses included a two within subjects factor repeated measures analysis of variance (Dose x Time) using SPSS for the microcomputer. Due to large expectancy effects which occurred at the end of the session, the two-hour time point for all variables not included in the analyses. Statistical significance was defined as a p-value equal to or less than a five percent probability (P ≤ 0.05) of a chance occurrence.
F. Results: Eighteen male cigarette smokers who were required to be abstinent prior to receiving the drug infusions in sessions 3 and 5 participated. Upon receipt of the serum cotinine concentration data, four subjects were found not to be abstinent from cigarette smoking during the abstinence phases. Their data were excluded from subsequent statistical analyses. Of the four data sets removed, two received drug first and two received placebo first maintaining the counterbalanced- order design. The data presented herein represent those collected from the 14 study-compliant completing participants.
The participants were healthy male cigarette smokers whose average age was 25.6 years (SD=6.5). None of the participants were interested in cigarette smoking cessation. They smoked an average of 25.4 (SD=6.0) cigarettes per day. Their average expired-air carbon monoxide concentration was 9.1 (SD=7.3). Their average expressed-air carbon monoxide concentration was 28.1 ppm (SD=10.3). The average FTC estimated nicotine yield of their cigarettes was 0.87 (SD=0.3). Their average baseline serum cotinine concentration was 378 (SD=16.3). Their mean education level was 14.5 years (SD=1.7).
In order to determine the 48-hour tobacco abstinence period produced significant changes in tobacco withdrawal symptomatology, sessions 2 and 4 (ad libitum cigarette smoking) were compared with 3 and 5 (after 48 hours of abstinence). For the VAS measurements, significant increases in self-ratings of "Need for Cigarettes" (F[1,13]=29.8; p = 0.001), "Craving for Cigarettes" (F[1,13]=41.4; p = 0.001), "Craving for Tobacco" (F[1,13]=34.3; p = 0.001), "Down" (F[1,13]=15.2; p = 0.002), "Anxious" (F[1,13]=13.6; p = 0.003), and a significant decrease in "Pleasantness" (F[1,13]=22.5; p = 0.001) were observed as a function of time. The TWSC showed significant increases in self-ratings of "Craving for Nicotine" (F[1,13]=40.1; p = 0.001), "Irritable/ Angry" (F[1,13]=55.4; p = 0.001), "Anxious/Tense" (F[1,13]=53.0; p = 0.002), "Difficulty Concentrating" (F[1,13]=31.6; p = 0.003), "Restless" (F[1,13]=30.2; p = 0.001), "Impatience" (F[1,13]=22.9; p = 0.001), "Excessive Hunger" (F[1,13]=6.2; p = 0.03), "Insomnia" (F[1,13]=7.4; p = 0.02), "Increased Eating" (F[1,13]=14.3; p = 0.002), "Drowsiness" (F[1,13]=5.9; p = 0.03), "Headaches" (F[1,13]=9.1; p = 0.01), and the TWSC subtotal score (F[1,13]=63.3; p = 0.001) as a result of tobacco abstinence.
Other subjective parameters were also noted to change significantly as a function of tobacco withdrawal. Using the AEQ, significant increases in self-ratings of "Irritable" (F[1,13]=43.8; p = 0.001), "Restless" (F[1,13]=19.5; p = 0.001) and "Lightheadedness/Dizzy" (F[1,13]=4.5; p = 0.05) were found as a function of tobacco abstinence. With the POMS, significant increases for various upscale scores were observed including tension/anxiety (F[1,13]=31.5; p = 0.001), depression/dejection (F[1,13]=8.4; p = 0.02), anger/ hostility (F[1,13]=12.2; p = 0.004) and confusion (F[1,13]=6.5; p = 0.03) as a function of tobacco deprivation. Also, there was a significant decrease in the POMS vigor subscale score (F[1,13]=8.5; p = 0.02) as a result of tobacco abstinence. For the physiologic parameters, no significant change in blood pressure was found; however, heart rate (F[1,13]=31.2; p = 0.001) decreased significantly after 48 hours of tobacco abstinence.

**Table 1**

| **BIOCHEMICAL MEASURES** | | | | | |
|---|---|---|---|---|---|
| Variable | Cotinine Mean (SE) | Placebo Mean (SE) | Difference Mean (SE) | T-SCORE | P-value |
| Serum Cotinine Concentration (session change) | 430 (26) | -11 (2.3) | 441 (27) | 16.4 | 0.001 |
| | | | | | |
| Serum Nicotine Concentration (session change) | 0.1 (0.1) | 0.0 (0.1) | 0.1 (0.2) | 0.6 | ns |
| ns = non-significant | | | | | |

The average baseline serum cotinine concentrations for the sessions were as follows (ng/ml): Session 2: 378 (SE=43); Session 3: 48 (SE=5.8); Session 4: 308 (SE=24); and Session 5: 54 (SE=6.7). The average baseline serum nicotine concentrations (ng/ml) for sessions 3 and 5 were 0.4 (SE=0.2) and 0.2 (SE=0.2), respectively. In Table 1, the sessional changes in serum cotinine and nicotine concentrations are listed. These values represent the session end minus session beginning concentrations. Serum cotinine concentration increased by 430 ng/ml of serum in the cotinine condition and decreased 11 ng/ml in the placebo condition (T(13) = 16.4; p = 0.001). More importantly, the serum nicotine concentration showed no change during the session which rules out the possibility of unanticipated nicotine administration as the agent responsible for the reported subjective effects in this experiment. The observed change in nicotine concentration was consistent with the limits of sensitivity of the analyses (SEM = 1 ng/ml).

**Table 2**

| **DEPENDENT MEASURES** | | | | | | | | |
|---|---|---|---|---|---|---|---|---|
| Variable | 0 min Mean (SE) | 5 min Mean (SE) | 15 min Mean (SE) | 30 min Mean (SE) | 60 min Mean (SE) | Dose P-Value | Time P-Value | DosexTime P-Value |
| SEDATED (VAS) | | | | | | | | |
| Cotinine | 26 (6) | 28 (6) | 27 (5) | 30 (6) | 28 (4) | .02 | .05 | ns |
| | | | | | | | | |
| Placebo | 24 (3) | 33 (5) | 42 (7) | 40 (7) | 37 (7) | | | |
| | | | | | | | | |

| RESTLESS (TWSC) | | | | | | | | |
|---|---|---|---|---|---|---|---|---|
| Cotinine | 3.1 (.3) | 1.4 (.3) | 1.4 (.3) | 1.6 g (.4) | 1.6 (.4) | .05 | .001 | ns |
| | | | | | | | | |
| Placebo | 2.6 (.4) | 0.9 (.2) | 0.9 (.2) | 0.9 (.2) | 0.9 (.3) | | | |
| | | | | | | | | |

| RESTLESSNESS (AEQ) | | | | | | | | |
|---|---|---|---|---|---|---|---|---|
| Cotinine | 1.8 (.2) | 1.2 (.1) | 1.2 (.1) | 1.4 (.1) | 1.4 (.2) | .05 | .001 | ns |
| | | | | | | | | |
| Placebo | 1.9 (.2) | 1.0 (.0) | 1.0 (.0) | 1.1 (.1) | 1.0 (.0) | | | |
| | | | | | | | | |

| INSOMNIA (TWSC) | | | | | | | | |
|---|---|---|---|---|---|---|---|---|
| Cotinine | 1.2 (.3) | 0.1 (.1) | 0.1 (.1) | 0.1 (.1) | 0.1g (.1) | .02 | .001 | ns |
| | | | | | | | | |
| Placebo | 0.6 (.3) | 0.1 (.1) | 0.0 (0) | 0.0 (0) | 0.0 (0) | | | |
| | | | | | | | | |

| PLEASANT (VAS) | | | | | | | | |
|---|---|---|---|---|---|---|---|---|
| Cotinine | 41 (3) | 49 (4) | 46 (4) | 45 (5) | 40 (5) | .05 | .04 | ns |
| | | | | | | | | |
| Placebo | 44 (3) | 53 (3) | 55 (4) | 50 (4) | 51 (4) | | | |
| | | | | | | | | |

| ANXIOUS/TENSE (TWSC) | | | | | | | | |
|---|---|---|---|---|---|---|---|---|
| Cotinine | 3.2 (.4) | 2.1 (.4) | 1.8 (.3) | 1.7 (.3) | 2.1 (.4) | .02 | .001 | ns |
| | | | | | | | | |
| Placebo | 2.9 (.4) | 1.1 (.2) | 1.3 (.3) | 1.2 (.2) | 1.0 (.2) | | | |
| | | | | | | | | |

| TENSION/ANXIETY (POMS) | | | | | | | | |
|---|---|---|---|---|---|---|---|---|
| Cotinine | 17 (2) | | | 10 (1) | 12 (2) | .005 | .001 | ns |
| | | | | | | | | |
| Placebo | 14 (2) | | | 6 (1) | 6 (1) | | | |
| ns = non-significant | | | | | | | | |

Intravenous cotinine base administration compared to placebo had no effect on heart rate, blood pressure or the ECG intervals (e.g., PR, QRS and QT). In Table 2, the subjective ratings of mood and cigarette withdrawal symptoms are listed. Throughout the study, subjects rated themselves as feeling less pleasant (p = 0.05) and less sedated (p = 0.03), while simultaneously reporting feeling more anxious/tense (p = 0.02), more tension/anxiety (p = .001), more restless (p = 0.05), more restlessness (p = 0.05) and more insomnia (p = 0.02) as function of cotinine administration. The feelings of tension/anxiety and restlessness were rated on different instruments showing some degree of reliability for these measures.
More specifically, as shown on Table 1, the subjective ratings of mood and cigarette withdrawal symptoms are reported. For the VAS, continine produced decreased self-ratings of "Pleasantness" (F[1,13]=4.6; p = 0.05) and "Sedated" (F[1,13]=7.1; p=0.02) when compared to placebo. Using the TWSC, continine (compared to placebo) produced smaller decreases in self-ratings of "Restlessness" (F[1,13]=7.5; p = 0.02), "Anxiety/Tension" (F[1,13]=7.1; p = 0.02) and "Insomnia" (F[1,13]=4.5; p = 0.05). With the AEQ, continine again produced a smaller decrease in self-ratings of "Restless" (F[1,13]=4.8; p = 0.05) than placebo. Using the POMS, cotinine again produced a smaller decrease in self-ratings of anxiety/tension (F[1,13]=12.7; p = 0.004) compared to placebo.
Time as a main effect significantly influenced all of the subjective parameters. Also in Table 1, the effects of time on the subjective parameters influenced by cotinine administration are reported. On the VAS, significant decreases as a function of time were observed for self-ratings of pleasantness (F[4,52]=2.7; p = 0.04) and sedated (F[4,52]=2.5; p = 0.05). For the WSC, significant decreases as a function of time on self-ratings of restlessness (F[4,52]=25.2; p = 0.001), anxiety/tension (F[4,52]=16.7; p = 0.001) and insomnia (F[4,52]=6.3; p = 0.001) were found. Using the AEQ, decreases in self-rating of restlessness (F[4,52]=13.0; p = 0.001) over time were seen. With the POMS, significant decreases in self-ratings of anxiety/tension (F[4,52]=20.7; p = 0.001) over time were shown. No significant Drug by Time interactions were observed for these variables.
The statistical analyses performed on the various measures of craving using data from both experimental sessions yielded no significant differences. This was likely due to variability generated by the subjects in the third session. Therefore, to examine the effects of cotinine on craving, a reanalysis of the data comparing cotinine versus placebo on the maximum sessional decrease from baseline for the various craving measures was performed using only session 5 data. The results are summarized in Table 3. The "Craving for Tobacco" visual analog score (p = 0.02) and the average of all craving scales (p = 0.05) were significantly decreased in the cotinine condition as compared to placebo. The average of all craving scores was achieved using the WSC "craving for nicotine" score multiplied by 20 and then adding all of the scores and dividing this total by four. There was a consistent directional effect across all measures with the cotinine showing a greater influence than placebo.

**Table 3**

| **MAXIMUM CRAVING DECREASE SCORE** | | | | | |
|---|---|---|---|---|---|
| Variable | Cotinine Mean (SE) | Placebo Mean (SE) | Difference Mean (SE) | T-SCORE | P-value |
| Need for Cigarettes (VAS-1) | -24.8 (6.3) | -13.7 (2.7) | -11.1 (6.8) | -1.63 | 0.07 |
| | | | | | |
| Craving for Cigarettes (VAS-1) | -19.6 (4.7) | -16.3 (2.7) | -3.3 (5.5) | -0.59 | ns |
| | | | | | |
| Craving for Tobacco (VAS-2) | -25.6 (3.8) | -14.3 (3.1) | -11.3 (4.8) | -2.33 | 0.02 |
| | | | | | |
| Craving for Nicotine (TWSC) | -1.56 (.18) | -1.11 (.26) | -0.45 (.32) | -1.41 | 0.09 |
| | | | | | |
| Average of All Craving Scales (VAS-1, -2, TWSC) | -24.9 (2.8) | -17.4 (3.2) | -7.5 (4.2) | -1.77 | 0.05 |
| ns = non-significant | | | | | |

**Table 4**

| Variable | Placebo Score < Cotinine Score n/18 | Cotinine Score ≤ Placebo Score n/18 | P-Value |
|---|---|---|---|
| Craving for Nicotine | 8/18 | 10/18 | ns* |
| Irritable/Angry | 10/18 | 8/18 | ns |
| Anxious/Tense | 10/18 | 8/18 | ns |
| Difficulty Concentrating | 9/18 | 9/18 | ns |
| Restless | 8/18 | 10/18 | ns |
| Impatient | 9/18 | 9/18 | ns |
| Excess Hunger | 9/18 | 9/18 | ns |
| Insomnia | 2/18 | 16/18 | .001 |
| Somatic Symptoms | 2/18 | 16/18 | .001 |
| Increased Eating | 3/18 | 15/18 | .004 |
| Drowsy | 6/18 | 12/18 | ns |
| Headache | 3/18 | 15/18 | .004 |
| Total Score | 11/18 | 7/18 | ns |

| | | | |
|---|---|---|---|
| * ns = non-significant | | | |

In Table 4, for the various tobacco withdrawal symptoms, the proportion of the 18 subjects whose total withdrawal score on a given symptom during the placebo session was less than that of the cotinine session are reported (n/18). For example, for the symptom "craving for nicotine," eight out of eighteen subjects reported less craving for nicotine throughout the session following the placebo injection as compared to the cotinine session. The various symptom scores were calculated by totalling all of the ratings measured at intervals of 5, 15, 30, 60 and 120 minutes after receiving an injection for a given symptom. The score for the placebo session was then compared to that obtained from the cotinine session. These data show that cotinine produced better effects (the placebo score was greater than cotinine) in a significantly larger proportion of subjects for the symptoms of insomnia, somatic symptoms, increased eating and headache. Significance was shown using the binomial sign test.
G. Discussion: The purpose of the study was to determine whether intravenously administered (-)-cotinine base has significant pharmacologic activity in abstinent cigarette smokers. The data presented herein is the first demonstration that cotinine is a pharmacologically active compound which produces many subjective changes in humans without affecting cardiovascular activity. Further, while cotinine administration appeared to exacerbate certain symptoms of the tobacco withdrawal syndrome including restlessness and anxiety/tension, it appears to decrease other tobacco withdrawal symptoms including increased eating, somatic symptoms, headache and craving for tobacco.

The present results suggest that cotinine is pharmacologically active in abstinent cigarette smokers. Although cotinine exacerbates certain symptoms of the TWS, it appears that this agent can significantly decrease other symptoms of tobacco withdrawal, including craving for tobacco, headaches, somatic symptoms and increased eating. This is the first systematic demonstration of cotinine's ability to influence various symptoms of tobacco withdrawal. Consequently, cotinine may play an important role in the process of nicotine dependence.

## Claims

1. The use of cotinine or a pharmaceutically acceptable salt thereof to prepare a medicament effective to reduce or eliminate at least one of the symptoms of nicotine withdrawal or of tobacco withdrawal syndrome in a human, wherein the medicament is a unit dosage form containing from 30 mg to 1000 mg active ingredient, calculated as (-)-cotinine in the free base form.

2. The use of cotinine or a pharmaceutically acceptable salt thereof to prepare a medicament effective to reduce or eliminate at least one of the symptoms of nicotine withdrawal or of tobacco withdrawal syndrome in a human, wherein the medicament is in a form for administration in an amount of from 1.0 to 100 mg/kg human body weight/day, of active ingredient, calculated as (-)-cotinine in the free base form.

3. The use of cotinine or a pharmaceutically acceptable salt thereof to prepare a medicament effective to maintain abstinence from, or assist cessation of smoking or nicotine use, wherein the medicament is a unit dosage form containing from 30 mg to 1000 mg active ingredient, calculated as (-)-cotinine in the free base form.

4. The use of cotinine or a pharmaceutically acceptable salt thereof to prepare a medicament effective to maintain abstinence from, or assist cessation of smoking or nicotine use, wherein the medicament is in a form of administration in an amount of from 1.0 to 100 mg/kg human body weight/day, of active ingredient, calculated as (-)-cotinine in the free base form.

5. The use of one of claims 1-4, wherein the medicament is a tablet or capsule.

6. The use of one of claims 1-4, wherein the medicament is a transdermal delivery system.

7. The use of one of claims 1-4, wherein the medicament is a chewing gum.

8. The use of one of claims 1-4, wherein the medicament is an intraocular insert.

9. The use of one of claims 1-4, wherein the medicament is adapted to administer the cotinine by inhalation.

10. The use of one of claims 1-4, wherein the medicament is an aqueous solution of cotinine or a pharmaceutically acceptable salt thereof.

11. The use of one of claims 1-4, wherein the cotinine is (-)-cotinine.

12. The use of one of claims 1-4, wherein the cotinine is a (-)-cotinine salt.

13. The use of claim 1 or 2, wherein the symptom that is reduced or eliminated is weight gain or excess hunger.

14. The use of claim 1 or 2, wherein the symptom that is reduced or eliminated is craving for cigarettes, nicotine or tobacco.

## Patentansprüche

1. Verwendung von Cotinin oder eines seiner pharmazeutisch akzeptablen Salze zur Herstellung eines Medikaments für die Verringerung oder Beseitigung von mindestens einem der Symptome von Nikotinentzugs- oder Tabakentzugssyndrom bei Menschen, wobei das Medikament eine Einheits-Arzneiform ist, die von 30 mg bis 1000 mg wirksamen Bestandteil, berechnet als (-)-Cotinin in Form der freien Base enthält.

2. Verwendung von Cotinin oder eines seiner pharmazeutisch akzeptablen Salze zur Herstellung eines Medikaments zur Verringerung oder Beseitigung von mindestens einem der Symptome von Nikotinentzugs- oder Tabaksentzugs-Syndrom beim Menschen, wobei das Medikament in einer Verabreichungsform in einer Menge von 1,0 bis 100 mg/kg menschliches Körpergewicht pro Tag an aktivem Bestandteil, berechnet als (-)-Cotinin in Form der freien Base, vorliegt.

3. Verwendung von Cotinin oder eines seiner pharmazeutisch akzeptablen Salze zur Herstellung eines Medikaments zur Erhaltung der Abstinenz von, oder zur Unterstützung der Einstellung des Rauchens oder der Verwendung von Nikotin, wobei das Medikament eine Einheits-Arzneiform ist, welche von 30 mg bis 1000 mg aktiven Bestandteil, berechnet als (-)-Cotinin in Form der freie Base, enthält.

4. Verwendung von Cotinin oder eines seiner pharmazeutisch akzeptablen Salze zur Herstellung eines Medikaments zur Erhaltung der Abstinenz von, oder zur Unterstützung der Einstellung des Rauchens oder der Verwendung von Nikotin, wobei das Medikament in einer Verabreichungsform in einer Menge von 1,0 bis 100 mg pro kg menschliches Körpergewicht pro Tag an aktivem Bestandteil, berechnet als (-)-Cotinin in Form der freien Base, vorliegt.

5. Verwendung nach einem der Ansprüche 1-4, wobei das Medikament eine Tablette oder eine Kapsel ist.

6. Verwendung nach einem der Ansprüche 1-4, wobei das Medikament ein transdermales Verabreichungssystem ist.

7. Verwendung nach einem der Ansprüche 1-4, wobei das Medikament ein Kaugummi ist.

8. Verwendung nach einem der Ansprüche 1-4, wobei das Medikament ein intraokularer Einsatz ist.

9. Verwendung nach einem der Ansprüche 1-4, wobei das Medikament zur Verabreichung des Cotinins durch Inhalation angepaßt ist.

10. Verwendung nach einem der Ansprüche 1-4, wobei das Medikament eine wäßrige Lösung von Cotinin oder eines seiner pharmazeutisch akzeptablen Salze ist.

11. Verwendung nach einem der Ansprüche 1-4, wobei das Cotinin (-)-Cotinin ist.

12. Verwendüng nach einem der Ansprüche 1-4, wobei das Cotinin ein (-)-Cotininsalz ist.

13. Verwendung nach Anspruch 1 oder 2, wobei das Symptom, welches verringert oder beseitigt wird, Gewichtszunahme oder übermäßiger Hunger ist.

14. Verwendung nach Anspruch 1 oder 2, wobei das Symptom, das verringert oder beseitigt wird, das Verlangen nach Zigaretten, Nikotin oder Tabak ist.

## Revendications

1. Utilisation de la cotinine ou d'un sel pharmaceutiquement acceptable de la cotinine, pour préparer un médicament efficace en vue de réduire ou d'éliminer au moins l'un des symptômes du sevrage de la nicotine ou le syndrome du sevrage de tabac chez l'être humain, selon laquelle le médicament consiste en une forme à dosage unitaire contenant de 30 mg à 1000 mg d'ingrédient actif, calculé sous la forme de la base libre (-)-cotinine.

2. Utilisation de la cotinine ou d'un sel pharmaceutiquement acceptable de la cotinine afin de préparer un médicament efficace pour réduire ou éliminer au moins l'un des symptômes du sevrage de la nicotine ou le syndrome du sevrage du tabac chez l'être humain, selon laquelle le médicament se présente sous une forme pour administration en quantité de 1,0 à 100 mg/kg de poids corporel humain/jour, d'ingrédient actif calculé sous la forme de la base libre (-)-cotinine.

3. Utilisation de la cotinine ou d'un sel pharmaceutiquement acceptable de la cotinine afin de préparer un médicament efficace pour maintenir l'abstinence du fumeur, ou pour assister la cessation ou le sevrage du fait de fumer ou d'absorber ou d'utiliser de la nicotine, selon laquelle le médicament se présente sous une forme de dosage unitaire contenant de 30 mg à 1000 mg d'ingrédient actif calculé sous la forme de la base libre (-)-cotinine.

4. Utilisation de la cotinine ou d'un sel pharmaceutiquement acceptable de la cotinine afin de préparer un médicament efficace pour maintenir l'abstinence du fumeur, ou pour assister la cessation ou le sevrage du fait de fumer ou d'absorber ou d'utiliser de la nicotine, selon laquelle le médicament se présente sous une forme pour administration contenant une quantité de 1,0 à 100 mg/kg de poids corporel humain/jour d'ingrédient actif calculé sous la forme de la base libre (-)-cotinine.

5. Utilisation selon l'une quelconque des revendications 1 - 4, selon laquelle le médicament se présente sous la forme d'un comprimé ou d'une capsule ou gélule.

6. Utilisation selon l'une quelconque des revendications 1 - 4, selon laquelle le médicament se présente sous la forme d'un système d'administration transdermique.

7. Utilisation selon l'une quelconque des revendications 1 - 4, selon laquelle le médicament se présente sous la forme d'une gomme à mâcher.

8. Utilisation selon l'une quelconque des revendications 1 - 4, selon laquelle le médicament se présente sous la forme d'un insert intraoculaire.

9. Utilisation selon l'une quelconque des revendications 1 - 4, selon laquelle le médicament est adapté pour administrer la cotinine par inhalation.

10. Utilisation selon l'une quelconque des revendications 1 - 4, selon laquelle le médicament se présente sous la forme d'une solution aqueuse de cotinine ou d'un sel pharmaceutiquement acceptable de cotinine.

11. Utilisation selon l'une quelconque des revendications 1 - 4, selon laquelle la cotinine se présente sous la forme de (-)-cotinine.

12. Utilisation selon l'une quelconque des revendications 1 - 4, selon laquelle la cotinine se présente sous la forme d'un sel de (-)-cotinine.

13. Utilisation selon la revendication 1 ou 2, selon laquelle le symptôme qui est réduit ou supprimé consiste en une prise pondérale ou en un appétit excessif.

14. Utilisation selon la revendication 1 ou 2, selon laquelle le symptôme qui est réduit ou éliminé consiste en le besoin très important de fumer des cigarettes, le besoin très important d'absorber ou d'utiliser de la nicotine ou du tabac.
